# EUROPEAN PATENT SPECIFICATION

(11) **EP 0 631 467 B1**
(45) Date of publication and mention of the grant of the patent: **03.06.1998**
(21) Application number: 93906775.7
(22) Date of filing: 29.03.1993
(51) Int. Cl.: A01H 4/00

(54) **IMPROVED MICROPROPAGATION SYSTEM**
MIKROPROPAGATIONSSYSTEM
SYSTEME DE MICRO-REPRODUCTION AMELIOREE

(30) Priority: 27.03.1992 IE 920993; 27.03.1992 IE 920994
(43) Date of publication of application: 04.01.1995
(73) Proprietor: MicroCrop Limited, Denbighshire, LL19 8LL (GB)
(72) Inventor: LONG, Roger, Tullow, Co. Carlow (IE); PERRIN, Alan Philip, Castledermot, Co. Kildare (IE); PERRIN, John Philip, Rhyl, Clwyd (GB)
(74) Representative: Walsh, Michael Joseph
(86) International application number: IE9300017
(87) International publication number: WO9319587

(56) References cited:
- EP-A- 0 389 019
- WO-A-90/06058
- WO-A-91/01630
- WO-A-91/03929
- WO-A-91/15110
- Permx-Multiplant : "A new face, a new technique; plant tissue culture production research and development" Brochure, 1991 Wageningen

## Description

This invention relates to micropropagation. In particular, the invention is directed to certain improvements in micropropagation techniques enabling enhanced levels of production of tuber-forming micro-plants in particular, such as potato plants.

In the micropropagation of plants such as potatoes, it is known to nurture certified micro-plants on a nutrient substrate medium consisting of sugars with macro- and micro-nutrients gelled in agar. Development of micro-plants in this manner is acceptable for small populations, but since the nutrient media are subject to bacterial and fungal contamination, the risk of loss of growth groups following such contamination is such that micropropagation based on the else of these media is less satisfactory when large plant populations are in question. While contamination may be minimised by high standards of hygiene and sterilisation, the significant level of manual input required in customary micropropagation processes renders the maintenance of high standards difficult of achievement.

It is also known to grow micro-plants on such media by their placement in a growth chamber under temperature control and with exposure to light. Typically, the light is provided by ordinary "white" fluorescent tubes and does not include any photosynthesising component. The sole source of carbohydrate is the sucrose, while there is a high mineral content in the nutrients, which are typically formulated according to MURASHIGE and SKOOG. After several generations of growing in this way, aberrations develop such that when plantlets raised on sucrose are planted out, they are not able to transpire by photosynthesis in the normal way, in other words they are non-photoautrophic. They are therefore not responsive to sunlight in the manner of a normal plant. Thus, in order to meet the objective of aseptic or sterile growth of large populations of plants, the objective of providing plants that are capable of growing in normal manner when planted out at the end of the micropropagation process may be lost.

In PCT/GB90/01001, International Publication No. WO 91/15110, which corresponds to the preambles of method claim 1 and apparatus claim 7, a method of micropropagation is described in which a series of horizontal cuts are made through a mass of fairly straight stemmed plantlets growing from a gelled nutrient medium to obtain cut crop parts containing on average at least one tip or node per cut portion. At each cut, the crop parts are transferred in bulk, for example by air flow or gravity, to a fresh gelled nutrient medium, where they are planted by random scattering. Harvesting of propagules or microtubers from plantlets may also be achieved, by combing through the crop with a comb and separating the propagules or microtubers by producing relative movement of the comb away from the gelled medium. Random scattering is seen as a less than optimal manner of re-planting the cuttings and may result in variable density of growth on the fresh substrate, with consequent uneven plant development.

It is an object of the present invention to overcome at least some of the difficulties recited above and to provide an improved process for micropropagation, and in particular for micropropagation of plantlets such as potatoes, which addresses the problems identified.

According to the invention, there is provided a method of micropropagation comprising the steps of:
(a) growing a plurality of plantlets on a first nutrient substrate medium,
(b) effecting a cutting operation on the plantlets at least at their apices to provide a plurality of apex cuttings for assembly into an organised array for subsequent growth on a second substrate medium,
(c) enclosing at least some of said apex cuttings and said second substrate medium to define an enclosed region,
(d) exposing said enclosed region to high intensity light and a carbon dioxide enriched external atmosphere, optionally with temperature control, for a period sufficient to secure a predetermined degree of microplant growth within said enclosed region,
(e) supporting microplants grown within said enclosed region, for a single cutting operation, in at least one flanged substantially rigid base structure for accommodating a body of substrate medium, said body of substrate medium defining said second substrate medium (11), and
(f) dividing said microplants in said single cutting operation to provide plantlet portions for further growth,
characterized in that
said body of substrate medium defining said second substrate medium has differential water and nutrient retention properties at different locations within said body of substrate medium, and that the method also comprises the following further steps:
(i) forming said at least one flanged substantially rigid base structure to comprise folded-in flanges or lips extending along side edges of said base structure,
(ii) compressing edge regions of said body of substrate medium to provide compressed edge regions in which the density of the substrate medium is increased, and
(iii) receiving said compressed edge regions within and under said folded-in flanges or lips of the base structure for gripping engagement of the body of substrate medium within said base structure.

The dividing operation of step (f) may provide plantlet portions, each of which comprises an apex and a plurality of nodes for planting out in a substantially natural environment for said further growth.

In a preferred elaboration of the method of the invention, the dividing operation of step (f) provides plantlet portions, each of which is an apex cutting, and said microplants and said second substrate medium are re-enclosed to again define said enclosed region for steps corresponding to steps (d) through (f) as recited above.

Suitably also, in the method of the invention in a favoured sequence, said apex cuttings provided by the dividing operation of step (f) are used for subsequent growth and division in steps corresponding to steps (b) to (f) as recited above.

In a still further elaboration of the method of the invention,
(1) said first nutrient substrate medium may comprise macro- and micro-nutrients gelled in agar,
(2) said cutting operation of stage (b) may provide a plurality of nodal cuttings and a plurality of apex cuttings, and
(3) at least some of said nodal cuttings are then placed in a further substrate medium comprising macro- and micro- nutrients gelled in agar for regrowth to provide further plantlets for use in a step corresponding to step (a) as recited above.

In the method of the invention, the cuttings are suitably assembled for said subsequent growth on a structured low density expanded polyurethane foam defining said substrate medium having differential water and nutrient retention properties.

Said low density expanded polyurethane foam is compressed and optionally restructured along said edge regions of the substrate, to provide said differential retention properties. The foam substrate is accommodated within said flanged substantially rigid base structure, the flanges of the base structure serving for retention of the foam material by gripping said side or longitudinal edges of a body of the foam material. The base structure may be perforated to provide for water circulation in the micro-environment within said enclosed region. The base structure is preferably perforated in a floor section thereof extending between said flanged side edges of the base structure. Said enclosed region is suitably defined by a material which is permeable to gas and vapour. Said material is suitably also transparent or translucent or otherwise light-transmitting for exposure to light of the growing plantlets. Preferably, a plastics enclosing material is used, thereby providing a readily pliable membrane to define the boundaries of the enclosed region. Suitably therefore, an elongate array of micro-plants is accommodated on said foam substrate, the substrate being retained within an elongate flanged base structure or container or tray as previously defined. Said array of micro-plants thus defines what may be referred to as "a hedge".

Thus in a favoured manner of application of the method of the invention, the cuttings are assembled for said subsequent growth on a structured low density expanded polyurethane foam defining said substrate medium having differential water and nutrient retention properties.

The cutting steps of the method of the invention may be carried out by inverting the micro-plants and severing the apices so that these fall away by gravity for collection and subsequent placement for further growth.

Alternatively, the hedge may be cut with the microplants or plantlets directed upwardly, in other words in a normal orientation. In either cutting circumstance, apparatus for effecting the cutting steps of the method of the invention suitably comprises means for constraining said array, i.e. the array defining the hedge, against significant displacement in at least one direction during said single cutting operation.

The invention also extends to apparatus for effecting a dividing operation in a method of micropropagation in which:
(a) a plurality of plantlets are grown on a first nutrient substrate medium,
(b) a cutting operation is effected on the plantlets at least at their apices to provide a plurality of apex cuttings for subsequent growth on a second substrate medium,
(c) at least some of said apex cuttings and said second substrate medium are enclosed to define an enclosed region,
(d) said enclosed region is exposed to high intensity light, optionally with temperature control, for a period sufficient to secure a predetermined degree of microplant growth within said enclosed region, and
(e) microplants grown within said enclosed region are divided in a single cutting operation to provide plantlet portions for further growth,
the apparatus comprising:
(1) a cutting head,
(2) means for supporting said microplants for said single cutting operation in said dividing step (e), and
(3) means for effecting displacement of said cutting head relative to said microplants, wherein said means for supporting said microplants for said single cutting operation comprises at least one flanged substantially rigid base structure accommodating a body of substrate medium, said body of substrate medium defining said second substrate medium,
characterized in that
(i) said at least one flanged substantially rigid base structure comprises folded-in flanges or lips extending along side edges of said base structure,
(ii) said body of substrate medium has differential water and nutrient retention properties at different locations within said body of substrate medium,
(iii) said body of substrate medium has compressed edge regions in which the density of the substrate medium is increased, and
(iv) said compressed edge regions are received within and under said folded-in flanges or lips of the base structure for gripping engagement of the body of substrate medium within said base structure.

The apparatus of the invention preferably comprises means for constraining said microplants against significant displacement in at least one direction during said single cutting operation. Said constraining means suitably includes at least two toothed comb-like structures for engaging portions of plant material forming an array of said microplants from opposite sides of said array. Each of said comb-like structures may be displaceably mounted for movement between array-constraining and array-release dispositions. Alternatively, and preferably, each of said comb-like structures may be an independent component for placement in an array-constraining disposition prior to said single cutting operation.

Thus, in an especially favoured arrangement, two said freely locatable toothed comb-like structures are provided for engaging said microplants from opposite sides of said array, for placement in an array-engaging disposition by an operator or automatically, prior to a dividing operation. By providing an arrangement of the foregoing kind, the cut portions of the array of microplants are held together by the teeth of the combs during the cutting operation and remain held in this manner following completion of cutting. Because the freely locatable combs are independent components removable from the cutting equipment, the entire cut-off mass of cuttings may then be removed, together with and still engaged within the combs, as a single entity, for subsequent separation of the cuttings from the combs and from each other at a further station of the micropropagation system. This further station may be a station where the cuttings are individually put in position in a further growth medium for development to define a further hedge or growth body, or a planting-out location where microplants are transferred to a substantially natural environment for further development.

Use of the method of the invention rapidly provides a substantial body of micro-plants of high quality, which are photoautrophic and develop in normal manner when planted out. Only one cut is taken from each hedge in each cutting operation. The hedge may then be allowed to grow for a further period of for example ten days, before a further cutting operation is carried out. It has been found that this repetitive cutting cycle may result in the development of micro-tubers in the root structure of the plantlets, where potatoes are in question, after a certain number of apex cuts have been taken. The reason for this development is not fully understood. It may arise from stressing of the plant, effected by the repeated cutting of the apices, or alternatively, it may arise from the increased age of plants in the hedge structure, as compared with the plant growth life more usually customary in micropropagation.

The invention will now be described in more detail having regard to the accompanying drawings, in which
Figure 1A and 1B together set down the various steps carried out in application of the method of the invention, also illustrated in diagrammatic pictorial representation,
Figure 2 is a pictorial representation of an initial stage of the method of the invention, in which apex and nodal cuttings are grown on gelled agar within a closed environment,
Figure 3 shows the separation of nodes and apices from fully grown microplants provided by the step illustrated in Figure 2,
Figure 4 is an enlarged representation showing the placement of an apex cutting in agar, for the step of Figure 2,
Figure 5 is a diagrammatic sectional view of a body of foam substrate for use in a further stage of the method of the invention, preparatory to the foam's being mechanically restructured to accept apex cuttings for further growth,
Figure 6 is a diagrammatic sectional view of the foam substrate of Figure 5, following a foam compression operation applied to longitudinal side edges of the body of substrate,
Figure 7 is an end sectional view of the modified body of substrate of Figure 6, as folded for insertion within a tray base for use in apex growth in the method of the invention,
Figure 8 is a pictorial representation of a section of a base material used to accommodate the substrate of Figure 7 for the phase of the invention in which apex cuttings form a so-called hedge,
Figure 9 is a pictorial representation of the substrate of Figure 7 in position within the base of Figure 8,
Figure 10 is a pictorial representation showing a multiplicity of apices following placement in the foam substrate of the arrangement of Figure 9,
Figure 11 is a pictorial representation of the apex array of Figure 10 as enclosed within a gas and vapour permeable membrane for growth to provide a hedge according to the invention,
Figure 12 is a pictorial representation of the array of Figure 11, as enclosed within a gas and vapour permeable transparent plastics material, in position for exposure to light to provide a high photo photon flux (PPF) ambience for hedge growth,
Figure 13 shows in diagrammatic form, a number of trays according to Figure 11 in position on racks in a growth chamber for exposure to high PPF light under an enhanced CO₂ regime and with temperature control, for the growth step of the method of the invention,
Figure 14 is a pictorial representation of a substantially fully grown hedge, preparatory to the severing of apices for further growth,
Figure 15 is a diagrammatic end view of the enclosure used in the hedge growth step of the method of the invention, illustrating the micro-environment within the enclosed region,
Figure 16 is a diagrammatic pictorial representation of a manner of separation of apices from a hedge grown according to the invention, in which the hedge is in an inverted disposition,
Figure 17 is a partial pictorial detail of an alternative embodiment of constraining member for use in the arrangement of Figure 16,
Figure 18 is a diagrammatic end view showing an array of micro-plants in position in the apparatus of Figure 16 preparatory to cutting, with constraining members of the kind shown in Figure 17 in position,
Figure 19 is a fragmentary top view of a dividing arrangement using a pair of contrarotating circular blades for the apex separation step,
Figure 20 is a similar schematic representation of an alternative and preferred manner of apex separation, in which the hedge is in an upright disposition,
Figure 21 shows a modification of the cutting procedure, in which removable combs are provided to retain the microplants in position during the cutting step and to facilitate removal of the cuttings following the dividing operation as well as providing a safety guard for rotating blades, and
Figure 22 shows further detail of the removable comb structure of Figure 20.

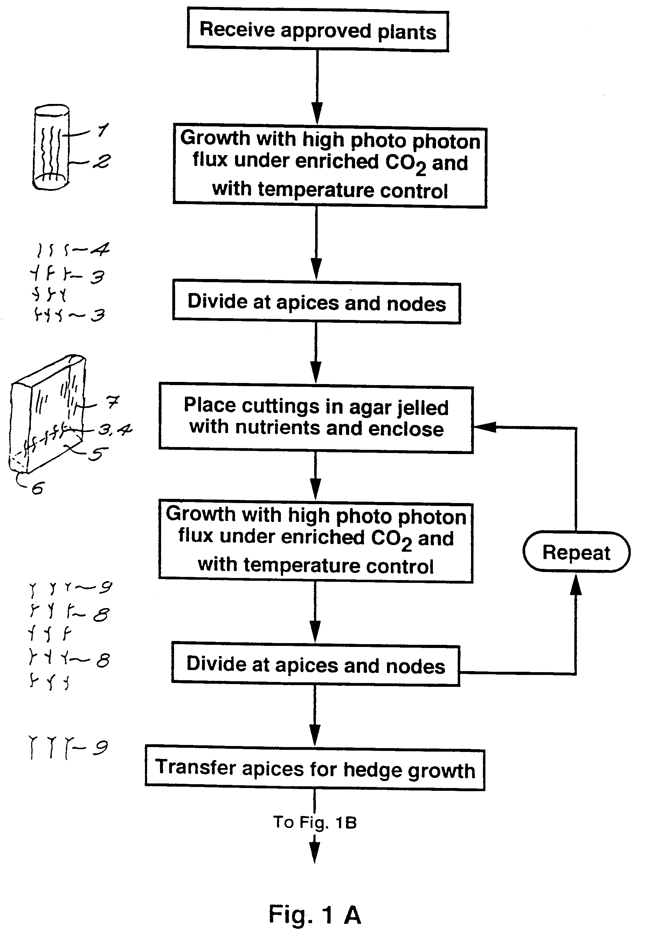

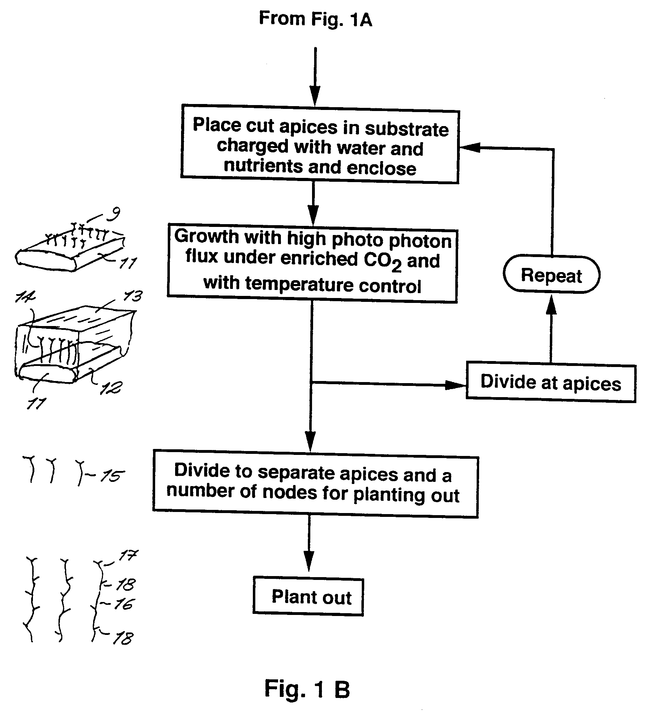

In application of the present invention, as shown in diagrammatic sequence in Figures 1, a group of standard approved plants 1 are initially received from an approved source. These plants 1 are typically received in standardised tubes 2, each tube typically containing some ten plants, and growth of the plants 1 within these tubes 2 takes place on a sugar base. In application of the method of the invention, these approved plants 1 are initially placed in an environment in which the level of carbon dioxide is enhanced and in which also the plants 1 are exposed to a high photo photon flux (PPF) by virtue of exposure to radiant light tubes providing a high density of ultraviolet light. The plants 1 are exposed to alternating cycles of high intensity light and carbon dioxide enriched atmosphere, between which periods the lighting is reduced to a normal level, sufficient to provide good working conditions for staff tending the plants, while also during these intervening periods, the carbon dioxide level of the local atmosphere is also reduced. The combination of exposure of microplants derived from nodal and/or apex cuttings to high intensity light of appropriate wavebands to establish a high photo photon flux density (PPF) with a broad spectrum of light wavelengths substantially equating to sunlight in conjunction with an atmosphere enriched with carbon dioxide is also a feature of the method of the invention in all of its various stages, optionally also with temperature control, in order to engender development of microplants capable of photosynthesizing when planted out under normal ambience.

Initial treatment of the standard plants 1 in the foregoing manner, typically for a period of for example ten days, enables the plants to become acclimatised to these high intensity light and carbon dioxide enhanced conditions, while continuing to grow on their original sugar base. At the end of this initial period, the plants 1 are removed from the tubes 2 in which they were initially received and are divided between nodal points, so as to provide a plurality of nodal 3 and apex 4 cuttings for further planting in a suitable support medium for further growth. The withdrawal of the plants from the initial tubes and the cutting operations which are carried out upon them are conducted under highly sterile conditions, all of the cutting and handling implements, such as scalpels and tweezers, being sterilised in an autoclave before use, and the cutting operations themselves being preferably carried on in a laminar flow cabinet. Operatives engaged in the tasks in question wear facemasks and surgical gloves, to ensure that the risk of contamination of the cuttings 3, 4 is minimised as far as possible.

The nodal 3 and apex 4 cuttings taken from the initially received plants are place in an agar gell 5, also comprising macro- and micro-nutrients, the gell 5 being supported within a suitable tray structure 6 enabling easy placement of the cuttings 3, 4 and providing space for subsequent growth of further microplants from the cuttings. In a favoured arrangement, to be further described, the agar gell 5 forms a triangular cross-section nutrient substrate medium portion in one end corner region of a substantially rectangular small tray 6. The cuttings 3, 4 are set into this non-flowable set gell, which is nonetheless sufficiently yielding to receive the cuttings in such a manner that the stem or shoot of each cutting extends substantially in elongate manner into the gell 5. Nodal 3 and apex 4 cuttings are preferably however not mingled, so that a particular tray 6 may contain apex cuttings 4 only or nodal cuttings 3 only, but not a mixture of apex 4 and nodal 3 cuttings.

Following placement of the apex 4 and nodal 3 cuttings, the tray 6 is wrapped within a gas and vapour permeable transparent plastics material 7, which is then suitably sealed to define a substantially closed environment within the plastics enclosure 7. The enclosed trays 6 are then stood on their ends in arrays for placement within again an environment in which the planted-out nodal 3 and apex 4 cuttings supported within the nutrient-rich agar gell 5 within the trays 6 may be exposed to a high photo photon flux (PPF) and carbon dioxide enrichment. When placed under this regime, with alternating periods of exposure to high intensity light and carbon dioxide interspersed with so-called "dark" periods, when carbon dioxide approaches normal atmospheric levels, very rapid growth of the cuttings 3, 4 takes place. For this stage of the method of the invention, no sugar whatever is used. Thus the plantlets develop in a manner substantially equating to that of normal plants, so that they are photoautrophic and capable at a later stage of the method of the invention of providing plants capable of continued normal growth when exposed to atmospheric air and sunlight, as compared with the impaired outdoor performance of plantlets originating from growth on sugar.

When the plantlets attain sufficient size, which takes place typically within a period of two weeks, at which time they have grown to occupy substantially the full elongate length or height of the tray 6 within which they are contained, the trays 6 are removed from the growth chamber in which they have been exposed to high intensity light as defined herein and an enriched carbon dioxide atmosphere, and further nodal 8 and apex 9 division takes place, to provide in first instance for further growth of plantlets in a repetition of the step just described, with a view to building up a critical mass of microplants originating with the originally approved source. When this critical mass is achieved, the apex cuttings 9, rather than being recycled to develop further microplants, are assembled for planting in a further medium for development into what is termed a hedge, i.e. a growth array. The cuttings 9 thus are now set for growth in an elongate structure 11 consisting of a low density expanded polyurethane foam material, adapted to provide differing water and nutrient potentials at different locations within the substrate structure, and the substrate 11 is itself accommodated in a flanged base member 12 (i.e. a tray or container), in a manner to be described in more detail subsequently. The tray base 12 has perforated holes and the substrate 11 is impregnated with water and nutrient. The tray 12, substrate 11 and plant products 9 are then all enclosed within a substantially transparent gas permeable plastics sheeting material 13, thereby again establishing a micro-environment within this enclosed space. Because of the characteristics of the substrate and the perforations in the flanged base 12, circulation of moisture and water vapour takes place within the enclosed environment inside the plastics sheeting 13. Moisture evaporates from the substrate 11 and the plantlets, condenses onto the plastics sheeting 13, flows down the inner sides of the enclosing plastics sheet 13 into the bottom of the enclosed region, and is then subsumed upwards again by the substrate 11 through the apertures or holes in the base or floor of the flanged member 12.

Growth of the micro-plants 14 in the hedge is engendered by placing the sheeted enclosed hedges within a carbon dioxide enriched external atmosphere under very bright light. The enclosing plastics sheeting 13 is substantially impermeable to liquid moisture, but is permeable to gas and vapour. The enriched atmosphere is therefore influential on the micro-environment within the enclosed region, which is nonetheless substantially closed to liquid moisture. Very rapid growth takes place. The hedge is defined by a series of plants 14 all of which are approximately the same vertical height. Cropping of rogue plants may be effected as necessary however to maintain this uniformity of height. When an appropriate measure of hedge growth has been achieved, the plant apices 15 are cut off, in a single operation, such as by inverting the hedge and effecting cutting in a manner described in co-pending applications and to be shown subsequently in the present application in representational form, in which a cutting means is traversed along the hedge in a cutting plane to sever the apices 15, which fall away by gravity for collection and subsequent re-planting. Alternatively, the single cut along the hedge may be taken with the hedge in an upright orientation, as again subsequently described in more detail. The cut-off apices 15 may then be rerooted for further growth and development, for establishing a further hedge. The original hedge is re-enclosed in plastics sheeting 13 as in the preceding stages, the substrate 11 resupplied with water and nutrient as required, and a further stage of growth of the original hedge takes place. Further apices 15 are thus formed or developed, and may again be cut off in a single further cutting operation in the manner already described.

When a sufficient number of hedges have been developed, cuttings 16 may be derived from them suitable for planting out in an external but protected environment, such as within so-called tunnels, where the plant 16 is exposed to daylight and atmospheric air, but within a sheltered environment corresponding substantially to a greenhouse. For this stage, the hedges are cut not merely to provide apex cuttings, but to yield cuttings 16 having an apex 17 along with a group of nodes 18. Thus in this final stage of hedge cutting, essentially the entire elongate length of the growing plantlet 14 is severed, near its base where it extends upwards from the substrate, so that typically four or five budpoints or nodes 18 are available. In planting out, the cutting 16 thus yielded is placed in soil or other growing medium with at least some of these nodal points 18 below the level of the soil, thereby providing for enhanced initial root and tuber growth, in the particular case of tuber forming plants such as potatoes.

It has been found that after several croppings, tubers may develop on the root systems of the plantlets within the substrate. These tubers may also be used, as micro-tubers, to be planted out and to form, for example, seedlings for a crop of seed potatoes. The reason for the development of the tubers is believed to be associated either with stressing of the plants occasioned by repeated cutting of apices, or alternatively by virtue of the plants within a hedge being relatively old, compared with the usual length of life of plants in micropropagation systems where intense nodal cutting takes place and a number of nodal cuttings are taken from each stem at each cutting operation. In these systems, plant life is necessarily reduced and the opportunity for re-growth is also reduced. However, the hypotheses indicated above are not necessarily definitive, and other reasons for tuber growth may also be present, but as yet unidentified.

The advantages described above are enhanced by use of a substrate such as is described inter alia in European Patent Specification No. 351,241A and a further related European Patent Specification, to provide the substrate medium used in the "hedge" stage of the method of the present invention. This substrate provides for different water potentials to exist at different locations within the substrate by providing by chemical formulation an initially hydrophilic unicellular foam, which is then modified by mechanical working to provide the differential properties noted. The substrate can therefore be adapted to provide any required degree of water holding and nutrient holding, by selection and mechanical modification of the substrate structure. In this manner, the necessity to provide the hedge or array within the enclosed region with any self-contained water supply and nutrient feeding system located within the region is avoided. Use of a substrate offering these features thereby not only facilitates the advantageous growth and micropropagation aspects afforded by the invention, but also greatly simplifies the manner of nurturing the enclosed plants during their enclosed phase of development.

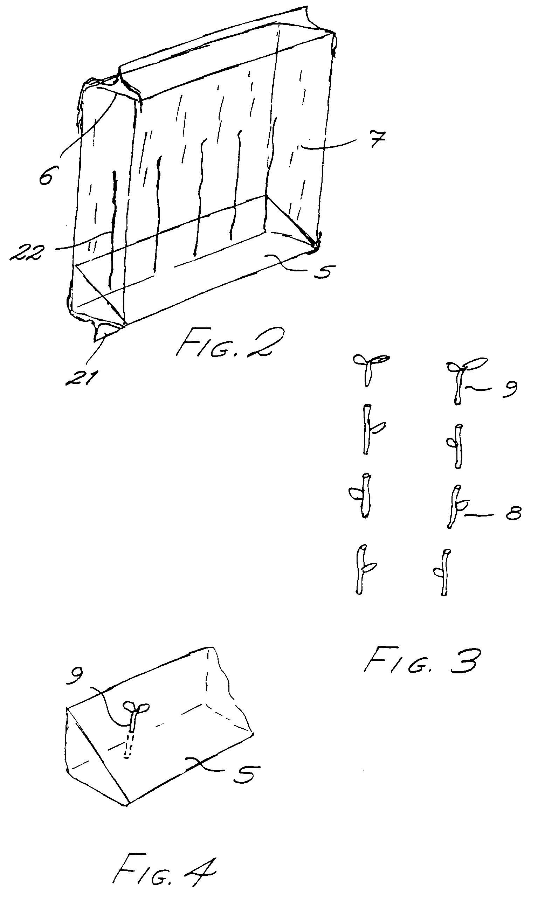

Figure 2 shows the manner of growth of apex and nodal cuttings on gelled agar 5 including macro- and micro-nutrients. As shown in the drawing, the agar 5 defines an elongate region of generally triangular cross-section in a lower corner of a rectangular or square tray 6, the triangular configuration being in no way a necessary feature but engendered by pouring a quantity of liquid agar and nutrient mixture into the tray 6, when the tray is in an initially tilted disposition. The liquid agar flows into the corner of the tray and sets. Thus the solidified agar gell 5 retains the triangular cross-section. The solidified agar 5 is however sufficiently yielding to accommodate the stems of shoots of the cuttings, which, following severance, are located by tweezers within the gell 5. The tray 6 is then enclosed and sealed 21 in the gas and vapour permeable plastics material 7, for placement in the temperature-controlled, carbon dioxide rich and high photo photon flux environment, in which growth of plantlets 22 is engendered. During its time in this ambience, temperature, carbon dioxide enrichment and light levels are all controlled in a cyclical manner.

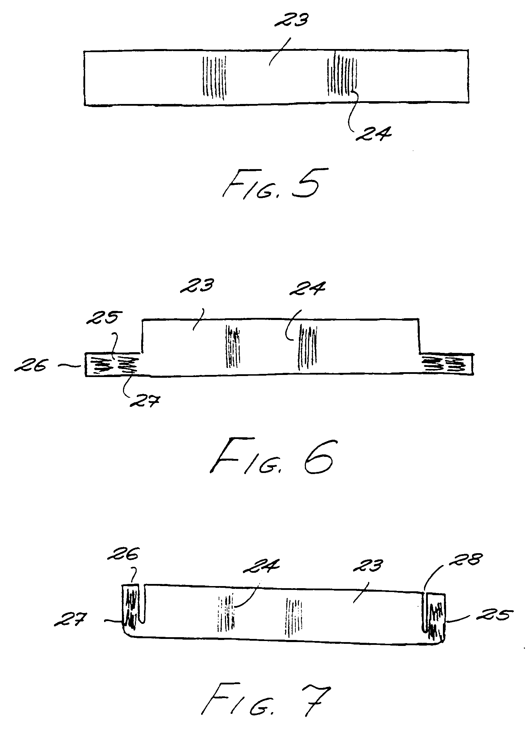

Figure 3 shows separated apex 4 and nodal 3 cuttings, following completion of the growth phase provided by the arrangement of Figure 2. Typically between four and five apex 4 and nodal 3 cuttings are retrieved from each plantlet 22, so that the multiplication rate is therefore typically between four and five, typically averaging at least 4.5, and on occasion approaching five.

Figure 4 is a pictorial representation of placement of a nodal cutting 3 in agar 5 for a further growing cycle in the environment of Figure 2. The shoot 9 is placed in a generally vertical orientation into the yielding gell using a tweezers. As previously explained, this process is continued until a sufficient number of plantlets have been established to provide a sufficient number of apex cuttings for placement in foam substrate for the next stage of the micropropagation method according to the invention.

Figure 5 is a sectional view of a block 23 of substrate 11 of the kind provided according to the abovementioned European Patent Specifications, as cut to substantially rectangular shape and approximate size from a larger volume of foam produced by mixing and foaming appropriate chemicals. This substrate block 23 has an open pore or grain structure, oriented in a vertical direction 24. Thus the grain structure 24 is aligned with the minimum thickness dimension of the block 23 of foam 11, that extending vertically upwards.

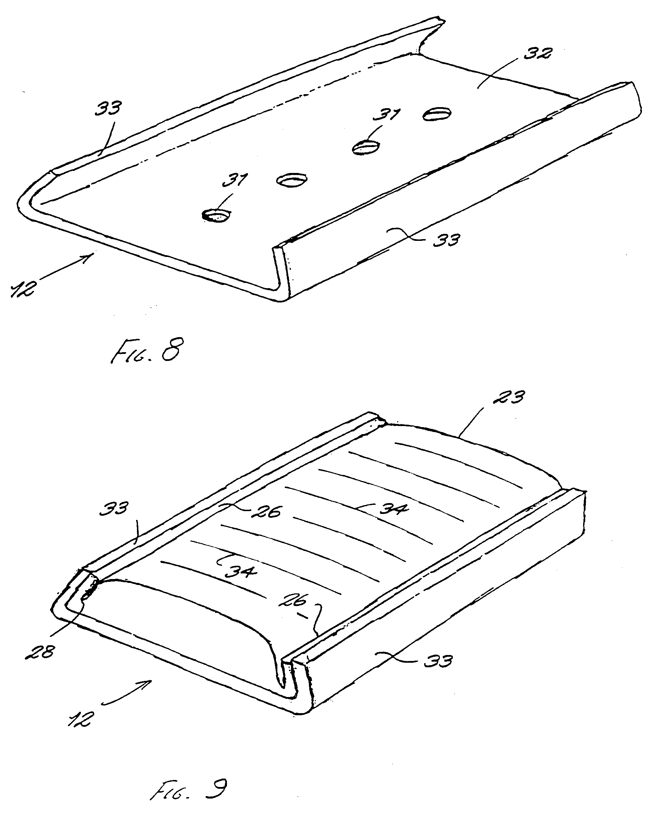

Figure 6 shows the next stage in preparing the foam substrate 11, 23 for use in the micropropagation method of the invention, in a preferred procedure, in which the block 23 of foam or substrate 11 of Figure 5 is compressed 25 along its longitudinal edges 26, so that the pore or grain structure 24 is caused to collapse from the vertical open tubular pores initially provided, which resemble the structure of plant cellulose, into a compressed or concertina form structure 27, as shown in diagrammatic representation in the sectional view of Figure 6, in these compressed side regions 25, 26. In this way, the density of the foam 11 is increased in these edge regions 25, 26, while its ability to retain water is enhanced, as compared with the open pore or loosely-grained structure 24 of the central region of the block 23, where the structure of the foam, already open in nature by virtue of the manner of its chemical formation, is also further opened up by mechanical perforation before the present compression or deformation for use for plant growth, in the manner described in the abovementioned European Patent Specifications.

Referring now to Figure 7, the mechanically-compressed edge 26 of the perforated open pore substrate 11 is then folded by pivoting or swinging these edge portions 25 upwards in hinge manner about their point of connection to the remainder of the substrate structure 23, and the substrate 11 is then engaged within a flanged base member 12, having upwardly directed side flanges which grip the upwardly folded compressed edge regions 25, 26 and hold the block 23 of foam substrate 11 firmly in position within the base 12. In this manner, as subsequently further described, an elongate block 23 of foam material 11 is firmly gripped within the base member 12, for the placement of apex cuttings 9, 15 for further growth in a subsequent stage of the micropropagation method of the invention. This gripping engagement may be facilitated by the external width dimension of block 23 being somewhat greater than the internal width of base 12, while also the upwardly-folded side edge regions 25, 26 of the block 23 are convexly dished somewhat in the outwards direction in the practical structure as compared with the idealized representation of Figure 7.

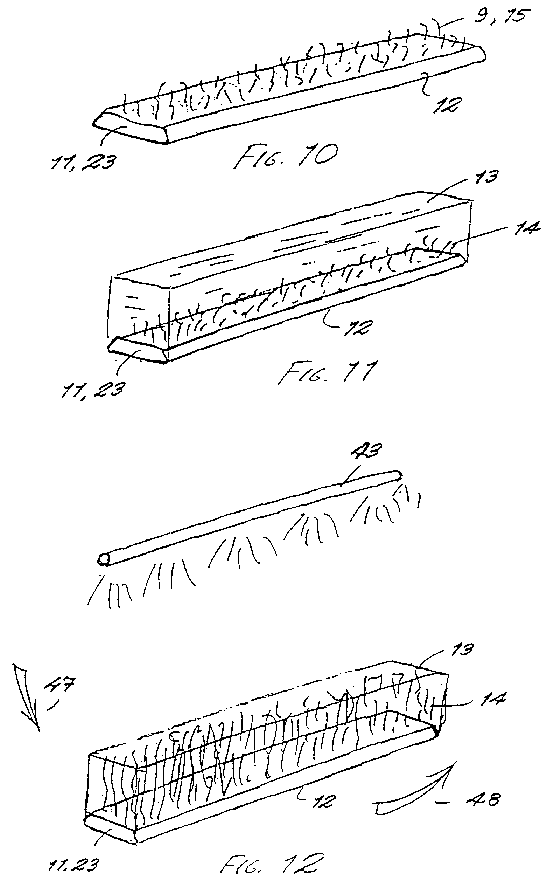

Figure 8 shows the base 12 in which the substrate 11 is accommodated. Figure 9 shows the base 12 and a section 23 of substrate 11 when accommodated within the base. As shown in Figure 9, base 12 is a flanged substantially rigid elongate structure. A series of central holes 31 in the floor 32 of base 12 provide for passage of water through the base 12, typically in an upwards direction for absorption in the foam structure 11. The foam 11 is formed by the aforementioned chemical expansion following a mixing operation, and in order to define a structure suitable for accommodation in the flanged base 12, the foam is then cut into a suitably dimensioned generally elongate strip 23 and subjected to compression 25 along its edges 26 to provide a compressed edge 27 on each longitudinal side, in the manner shown in Figure 9. This compressed edge 27 is received under the folded-in flange or lip 23 extending along the side edges of the elongate base 12. The foam edge 26 is preferably doubled over in the region of the base flange 33, as indicated by reference 28 in the drawings and as described above, this facilitating the absorption of water and nutrient and contributing to the establishment of the differing water and nutrient potential in the foam structure. The prepared substrate, mounted in the base member, is then charged with water and nutrients in an appropriate mixture and in predetermined quantity before placement of plant cuttings.

In achieving this feature, the vertical grain structure of basic foam is thus densified by heat and pressure along the edge region to give a higher level of water retention. Mechanical perforation to break up the cell structure further contributes to differential density. Perforations provided in this way allow for drainage and the passage of water, while the compressed cells at the side edges enable water absorption. The folded side edges pull water out from the centre of the substrate, when the substrate is saturated. Thus the side edges have a high water potential and the centre has a low potential. In this way the required circulatory micro-environment may be provided. In the present context, the "centre" of the substrate is its axially central portion in the elongate direction of the substrate strip or block.

When compressed by the application of pressure and heat, the same foam substrate may also be adapted to provide a so-called "chitting" substrate, suitable for the growth of plantlets for direct use, in which penetration of the substrate by the plant root structure is to be avoided. This "chitting" substrate has a greatly enhanced density, and provides a substrate of substantially reduced thickness, as compared with the cross-sectional properties of the foam shown in Figures 5 to 7 and 9.

A further feature of the substrate 11 as shown in Figure 9, following preparation for the reception of apex cuttings 9, 15 for further growth, is the provision of transverse slits 34 at substantially uniformly spaced locations along the elongate length of the foam body 23. These slits 34 provide for ease of placement of apex cuttings 9, 15 during their transfer to the foam substrate 11. The cuttings 9, 15 may be positioned manually at substantially uniform intervals across the width of the foam structure 23 by an operative, who eases the sides of the slit 34 slightly apart and positions the apex cutting 9, 15 within the parted lips of the slit 34 using a tweezers, so that the cutting is firmly held in position in the liquid and nutrient-charged substrate when the lips are released. Hygiene is observed, and a laminar flow cabinet is preferably used, but the level of sterility required at this stage is significantly less than applies in the initial build-up of critical mass and in the separation of the first round of apex cuttings 9 for placement in foam substrate 11 for the initial establishment of a first hedge. In other words, all of the operations concerned with initial plant growth in agar and using micro- and macro-nutrients involve a higher degree of sterility than applies when the growth regime is transferred to the foam substrate, where the risk of bacterial and biological infection is significantly reduced, by virtue of the inability of the foam material to support such infestation.

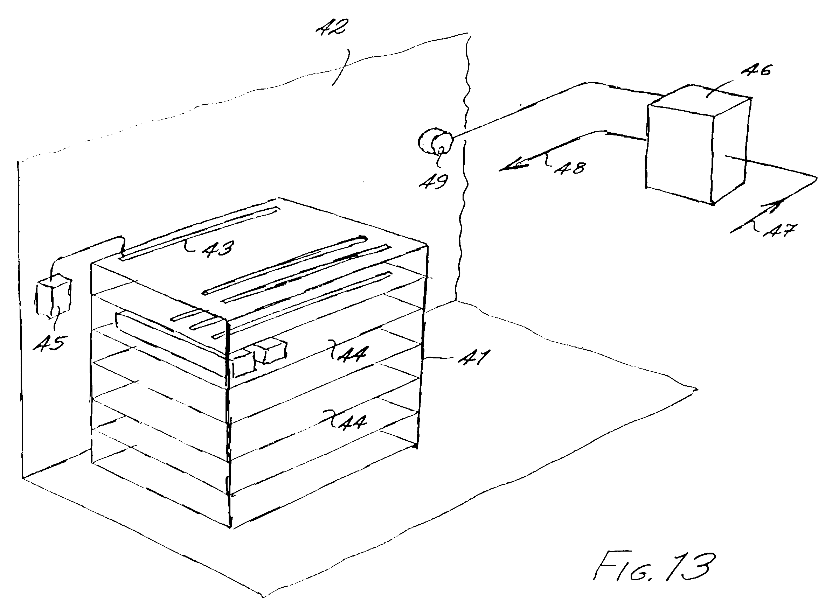

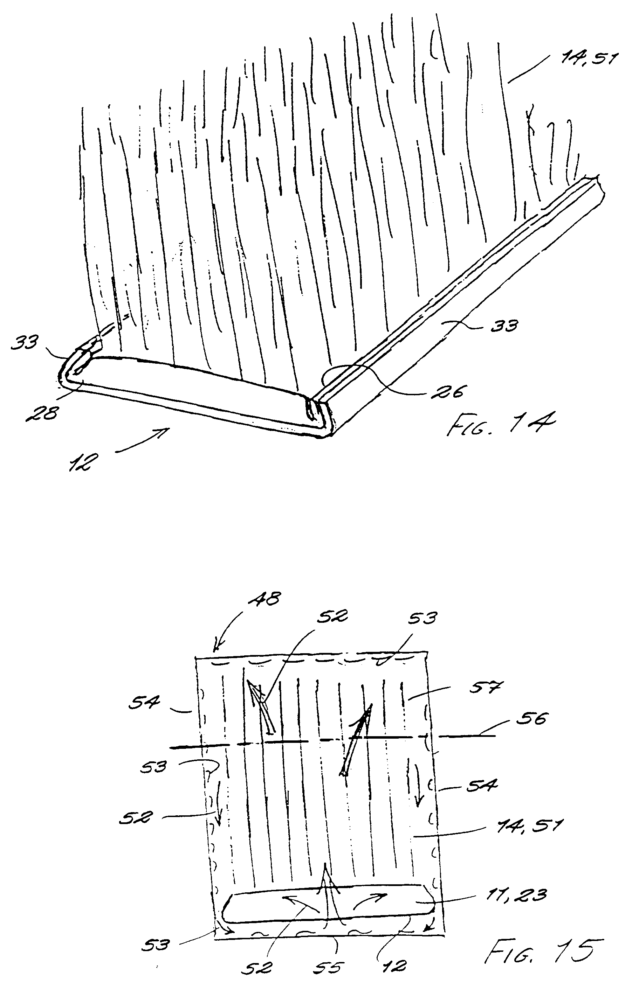

Figure 10 is a pictorial representation showing a series of apex cuttings 9, 15 following placement in foam substrate 11 held in an elongate base member 12, prepared in the above-described manner. As shown in Figure 11, these apex cuttings 9, 15 are then enclosed within gas and vapour permeable transparent plastics sheeting 13, in the manner previously described, to define an enclosed micro-environment. Referring now to Figure 12, the enclosed incipient hedge of cuttings, positioned in the foam substrate 11 and ready for growth, is then exposed to the environmental conditions previously described, namely cyclical exposure to enriched carbon dioxide external of the micro-environment, simultaneous exposure again on a cyclical basis to a high level of light (PPF), in other words high photo photon flux, together with temperature control as required. Figure 12 is a schematic representation of the ambience surrounding a single such tray 6, while Figure 13 is a diagrammatic representation of a system in which a large number of trays 6 are mounted in racks 41 to define arrays of trays within an environmental chamber 42, within which temperature, carbon dioxide level and light levels are all controlled and controllable, as indicated in the drawing.

Figure 12 shows a single gas discharge light tube 43, selected to provide a photo photon flux level of the required intensity and within a required specified spectrum. A multiplicity of these light tubes 43 are used in the rack arrangement shown in Figure 13, in which each level of the rack 41 is provided with a number of light tubes 43, to overlie at a short distance, trays 6 accommodated on the various shelves 44 of the rack structure 41. As far as possible, transparent, translucent or otherwise light-transmitting materials are used in the rack structure, and where use of light-opaque materials is unavoidable, these are preferably painted white for high reflectivity. Within the chamber 42, a time switch 45 controls the duration of exposure to light of the growing microplants. Apparatus designated generally by reference 46 provides for the maintenance of an appropriately carbon dioxide enriched atmosphere within chamber 42 by circulatory flow designated by references 47 and 48, under the control of a sensor 49, the enhanced CO₂ environment, which affects the microplants through the permeable enclosure 13, being of especial importance in achieving the ability of plants grown according to the invention to sustain "normal" growth when planted out. Features 46 through 49 may also serve for temperature control of the ambience within the growth chamber 42.

Figure 14 shows a hedge 51 grown according to the invention following establishment of plant growth. Uniformity and consistency of density of the micro-plantlets 14 is characteristic of the hedge or array 51 provided by the invention, this facilitating and contributing to the ease with which apex severance may take place, while yet enabling continued growth for further separation of further cuttings.

Figure 15 is a cross sectional end view of an enclosed hedge or array 51 according to the invention, illustrating the micro-environmental properties of the system of the invention. As shown in this drawing, the hedge 51 is accommodated within plastics sheeting 13, for upwards growth from substrate 11 retained in base tray or container 12. The paths of suspiration and evaporation of moisture contained within the micro-environment and initially supplied in the form of water, along with suitable nutrient materials, to the substrate or foam 11, are indicated by arrows 52, and this moisture condenses in the form of droplets 53, which build up or accumulate on the inner surfaces of the top and sides of the enclosed region defined by the plastics sheet 13. These condensed water droplets 53 run down the sides 54 of the plastics sheet 13 and accumulate on the plastics floor 55 of the enclosed region, underneath the floor 32 of the base member or tray 12. The accumulating water on the floor 55 is drawn upwardly again into the water absorbing and the water-retentive foam 11 through the holes or apertures 31 in the floor 32 of the base 12. In this manner, there is a continual recirculation of the water within the enclosed micro-environment, in a manner conducive to plant growth. These properties of the system of the invention obviate any necessity to provide any kind of controlled water feed and distribution system within the enclosed region. The properties of the foam structure 11 together with enclosure of the growth array or hedge 51 in themselves provide an appropriate micro-environment for hedge growth and development. These features of the invention thereby greatly simplify the development of micro-plants 14 to a sufficient stage of growth for separation of apices 15, by way of a single cut carried out along a cutting plane indicated in this drawing by reference 56. Pre-charging of the substrate 11 with water and nutrients is effected prior to plant cutting placement and enclosure, in sufficient quantity to maintain the micro-environment comfortably for the required period of growth. The properties of the substrate as placed in the base 12, with the compressed edge region structure 27, engenders a growth regime in which the greatest concentration of nutrients is located along the compressed edge regions, where the water-retentivity of the substrate is greatest. Nutrient conductivity is substantially lower in the centre of the substrate. This growth regime engenders initial safe but rapid growth of the plants in the low nutrient concentration central region, while providing more than adequate reserves of nutrient to sustain plant growth at the later stages, according as the plant becomes bigger and its root network extends out into the edge reservoir regions of the substrate, where nutrient concentration is higher.

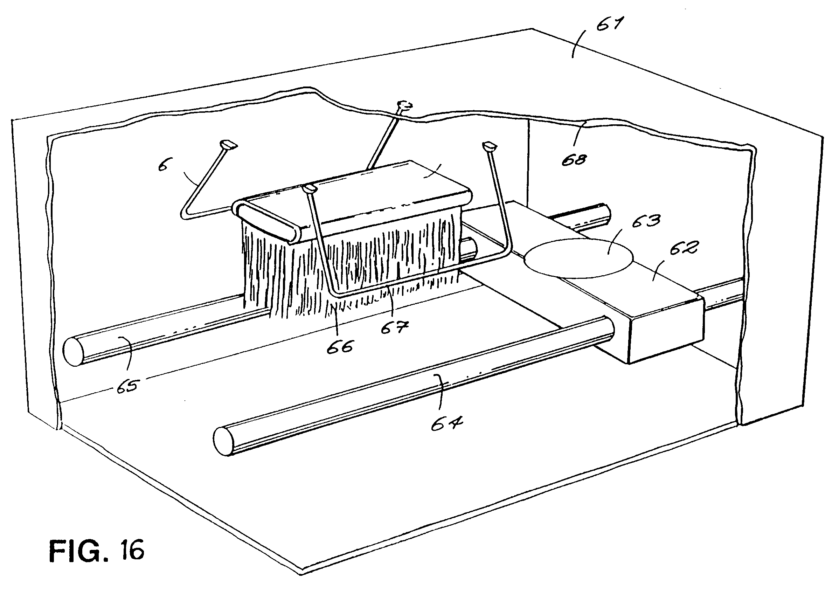

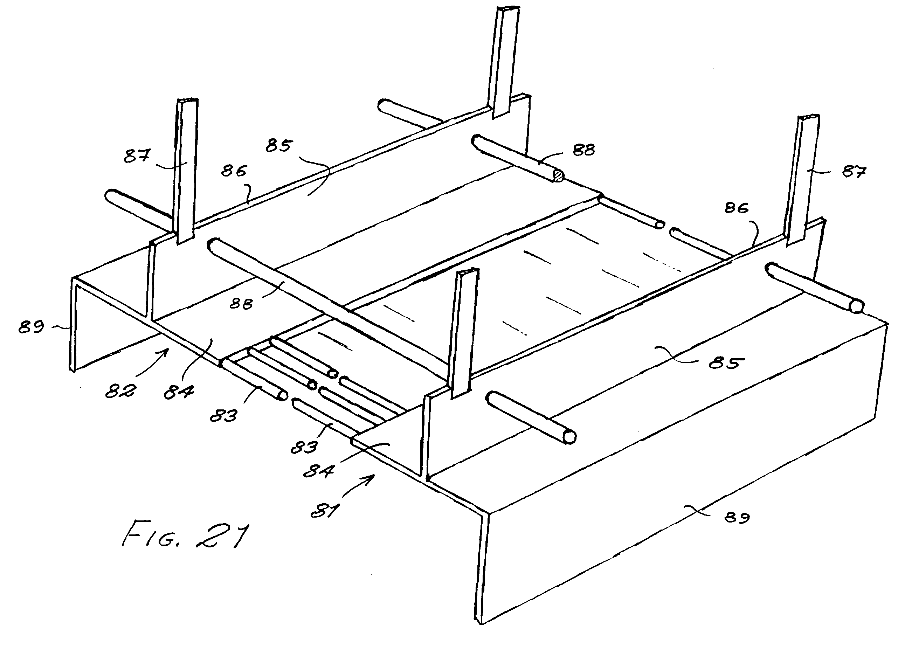

In application of a single cutting operation to the hedge 51 to produce apices or cuttings 15 for further growth, the hedge 51 may be oriented in an inverted disposition as shown in Figure 16, or alternatively, the cut may be taken with the hedge in the upright disposition, as shown in Figure 20. In either disposition, an engaging structure may be used to stabilise and grip the plantlets to be cut, especially where a rotary cutter is in question, as shown in Figures 16 and 20.

As shown in Figure 16, an enclosure 61 in which cutting takes place may be adapted for substantially contamination-free cutting, such as by the provision of a laminar-flow cabinet, or by sterilisation, using other means, of the internal environment within the enclosure. Such a feature or requirement is not however essential to the invention. The enclosure is provided with an arrangement, not shown in this diagrammatic pictorial representation, for holding an elongate tray 12 containing a multiplicity of microplants 14, in an inverted disposition. A suitable such arrangement may be provided by using laterally grooved trays 12 in conjunction with a support feature such as longitudinal ribs or ridges. A cutting head 62, provided with a rotary circular blade 63, is mounted for travelling displacement in a direction extending along the length of the elongate tray 12. Cutting head traverse takes place along two guide members 64, 65, suitably in a sliding manner. The rotary blade 63 is mounted on the cutting head 62, for rotation in a plane 56 (Figure 15), extending substantially parallel to a plane defined by the growth tips 66 of the microplants 14. The rotary blade 63 is driven in rotation at high speed by a drive motor accommodated in the cutting head or carriage 62.

In the arrangement shown, blade drive and cutter head travel are independent. In order to effect a cutting operation, the rotary blade 63 is set in motion, the relative placement of the growth tips 66 of the microplants 14 is adjusted relative to the cutting plane 56 of the blade 63 so that the growth apices 15 of the plantlets 14 will be cut off in the cutting operation, and the rotary blade 63 is then traversed along the length of the inverted tray 12, such as by manual displacement of carriage or cutting head 62, thereby severing the apices 15. The cuttings fall into a collection region at the base of the enclosure 61. According as cutting head 62 moves forward, it advances into a space freed by severance of the apex regions of the plantlets, so that the bulk of the cutting structure does not in any way impinge upon or damage either the hedge array or the cut portions.

Preferably however, carriage drive also is motorized, as well as blade drive. In an adaptation of the structure shown, cutting head traverse may take place by at least one of the guide shafts 64, 65 on which the cutting head 62 moves being also drivable in rotation, for drive by an external motor mounted outside enclosure 61. This rotatable shaft 64 or 65 may thus then serve as a lead-screw or like mechanism to effect cutting head 62 traverse in both directions, with appropriate features for reversal of drive at the end of the traversing motion. The second rotatable guide shaft 64 or 65 may also be powered to drive the blade 63 in rotation through a suitable gear train within the cutting head 62, is an alternative to a local motor within head 62. In an alternative construction, both drive features may be activated by a single rotatably mounted shaft, again driven from outside the enclosure.

In this cutter structure, the cutting takes place by virtue of the rotary action of the circular blade 63. The rotary blade 63 thus provides a single-element cutter as herein defined. The rapidly moving cutting edge of this blade 63 cuts through the plant material 14 as the blade edge passes at great speed across the plant stem 57 (Figure 15). In order to stabilise however the plantlet 14 or "hedge" 51 structure or array provided in the inverted tray 12, the apparatus of the invention preferably also comprises a plant-supporting structure, in the form of a pair of laterally displaceable bar or bale members 67, suitably supported from the roof 68 of the enclosure 61 by arms 69 pivotally mounted on and depending from roof 68. Bars 67 are located to the sides of container or tray 12 when mounted in an inverted disposition in enclosure 61, and are pivotable inwardly and outwardly relative to the plant array 51. Members 67 are moved inwardly therefore against the hedge 51 or plantlet 14 array to define and constrain side edges of the array 51 during a cutting operation. A suitable locking mechanism associated with the pivoting arms 69, such as an over-centre mechanism of known kind, may be provided to retain the bar members 67 in their constraining or inwardly displaced disposition. Similarly, when the bar members 67 are pivoted into their outward dispositions, the locking or over-centre mechanism associated with the pivotal mounting of arms 69 may hold the bar members 67 in this clear or inactive configuration, in which placement and removal of microplant trays or containers 12 is facilitated.

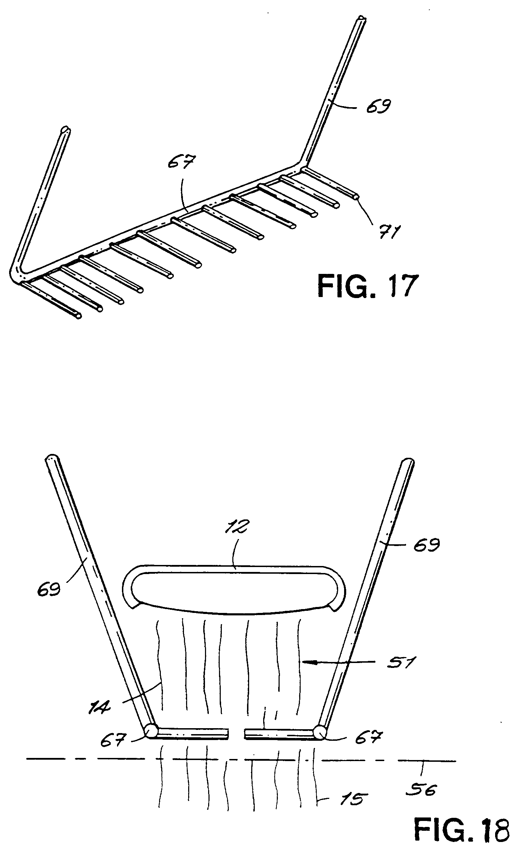

The lateral members 67 may also or alternatively be provided with a series of inwardly directed teeth 71, Figures 17 and 18, which engage the plantlets 14 inwards from the side of the container or tray 12. The teeth 71 approach one another towards the centre of the hedge or plant growth body 51, as shown in Figure 18, but do not however meet or interengage or overlap in a meshing manner in the construction shown, although such a construction may also be provided. Figure 18 shows the bar members 67 at the lower ends of the pivotal arms 69 in a co-operating plantlet-constraining or hedge-defining disposition, in which the arms 69 have been pivoted inwards so that the bar members 67 engage gently against the sides of the outermost microplants 14 in the array 51 of plantlets 14 held in the tray or container 12. The teeth 71 penetrate between microplants 14 of the array 51, and the inward ends of the teeth 71 of each bar member 67 are closely juxtaposed at the centre of the array 51, but do not meet, in the construction shown. Thus a series of generally rectangular zones are defined within the hedge or array 51, each zone having a boundary defined by portions of bar members 67 and teeth 71. This array of zones provides a multiplicity of bounded regions which constrain and support the plant array 51 for a cutting operation. In this manner therefore, the interleaving structure of the teeth 71 and the sidewise support of the bar or lateral members 67 ensure a high quality of cut, during separation of apices 15 along cut plane 56, Figure 18.

The high speed of rotation of the blade 63 is believed to establish a boundary layer in front of the blade, which may in certain circumstances tend to displace plants 14 away from the blade during blade traverse in a cutting operation. The supporting structure provided as described above overcomes however any tendency of plantlets to move away from the advancing and rotating blade. Alternatively, the rotary blade may be adapted on its lower face to establish a low pressure region beneath the blade, thereby counteracting any boundary layer action ahead of the blade tending to push the plant stem away by a counterbalancing pulling force beneath the blade.

The invention is especially effective in achieving sterile cutting. The high speed of rotation of the blade ensures that there is no significant accumulation of plant material on either surface of the blade. Alternative single-element cutters may however also be used, in particular non-physical cutters such as laser cutters.

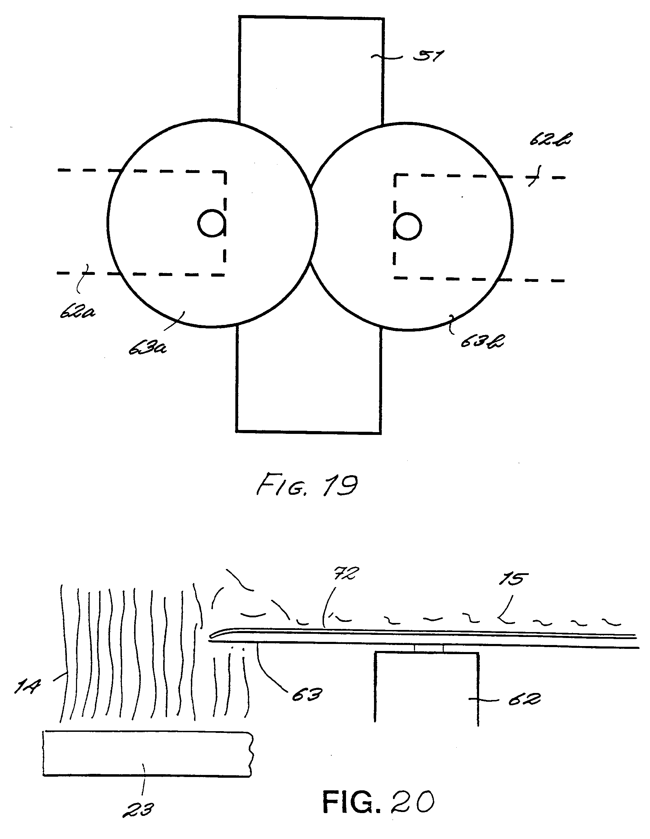

As described in Figure 16, a single rotary blade 63 only is used. Figures 19 shows in diagrammatic plan view an alternative construction in which two contra-rotating planar circular blades 63a, 63b are used. The blades 63a, 63b rotate in the opposite direction so that the blades thus rotate inwardly, and thereby tend to accentuate and contribute to the holding action of the bar members 67, where present, or to gather the hedge inwards, in the case of an unconstrained plant array. A slight overlap is provided between the cutting regions of each blade. This dual cutter structure also enables the cutter heads 62a, 62b incorporating the respective drive arrangements for the blades 63a, 63b to be located outside the spatial envelope corresponding to hedge array 51. Thus this blade arrangement is especially suited to upright cutting, where clearance for blade drive and support is less readily achieved for a single rotary blade.

In an especially preferred application of the method of the invention, upright cutting is however used, as shown in one exemplary form in Figure 20. In this instance, a sheath or cover 72 comprising a substantially planar member may be placed above the rotary blade 63. Cut plant nodes falling away from the hedge or plant array 51 are collected on this sheathing surface 72, and may be swept away and collected for replanting by suction or any other relevant suitable arrangement. A multiplicity of further possibilities then exist in regard to further handling of the cut plant portions, following collection after severance, for subsequent downstream placement for further growth and development, including air streaming or entrainment in carrying and conveying media of other kinds. The necessity for a compact cutter head 62 in order to avoid head contact with the uncut portions of plantlets 14 will also be apparent.

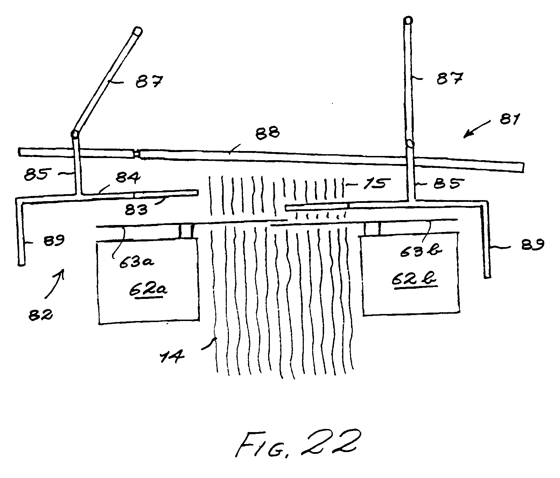

As shown in Figures 21 and 22, a removable stabilising comb structure may alternatively be used in the cutting step of the present invention. In this arrangement, two opposing comb members 81, 82 are pressed into the hedge 51 from opposite longitudinal sides in a plane above the cutting plane 56 in the normal or upright orientation of the hedge shown in Figure 22, in other words on the opposite side of the cutting plane from the root portions of the plantlets 14. The combs 81, 82 thus engage portions of the plantlets 14 to be cut off, namely the apices 15. The comb members 81, 82 have a toothed structure corresponding to that shown in Figure 17, but in this instance the teeth 83 extend from a planar generally horizontal flange portion 84. An upright fin 85 extends substantially vertically from flange 84 and is disconnectably supported at its upper edge 86 by pivot arms 87 receivable in sockets or like attachment points on the roof or in an upper region of the cutting chamber or location. Structures 81 and 82, each defined by teeth 83, flange 84 and fin 85, can be pivoted towards one another when suspended by arms 87, and are locked together with the tips of teeth 83 at a small spacing by means of transverse locking bars 88, which comprise pawl or like auto-engagement features latching into place when the removable combs are correctly in position in their closed-together condition. In Figure 22, comb 81 is shown in its final position and comb 82 in its initial outwardly-pivoted disposition.

In use of this arrangement therefore, arms 87 are engaged to support the structure 81, 82, the array 51 is placed in position for cutting, teeth 83 engage plantlets 14 in the apex region 15 above the cutting plane 56, and the combs 81, 82 are locked together by bars 88. Cutters 63a, 63b sever the plantlets along the cutting plane 56. On completion of the cutting operation, the cut portions 15 remain therefore engaged by the combs 81, 82 and may be readily collected by removing the combs 81, 82 from the hedge 51 and sliding out the engaged cut portions or apices 15 from their retained positions between the teeth 83 of the removable comb portions 81, 82. The combs or comb-like members are thus freely locatable and do not form an integral connected part of the cutting apparatus. The comb structures 81, 82 may actually comprise the arms 87, these then being attachable to the cutting environment, or alternatively, arms 87 may be part of the cutting chamber, and structures 81, 82 attached to them and detached from them as required.

This removable comb arrangement 81, 82 ensures that the cut portions are retained in an integrated sub-array or assembly following cutting, to facilitate easy collection for subsequent placement. The removable combs 81, 82 are effective irrespective of the orientation in which the cutting is carried out. Thus the combs may be applied to upright cutting, in the orientation of Figure 21, or alternatively to inverted cutting, such as is shown in Figure 16, in which the arrangement used represents a substantial inversion of that represented in Figure 22. The use of the removable comb arrangement 81, 82 greatly facilitates tidy and organised collection of cut portions for subsequent planting out and avoids the random disposition of cut portions which is inherent if there is no control or constraint on the cut portions or apices 15, while also avoiding cut portions becoming entangled in the remainder of the hedge, which occurs if upright cutting is applied without an appropriate cutting retention or collection feature.

In the comb arrangement 81, 82 described for engaging plants in a releasable manner, the comb structure, in addition to engaging the severed portions 15 of the plants 14, also provides a guard for the cutters during the cutting operation in the form of a downwardly extending further flange 89 located outward of the outer edge of the blade 63a or 63b in the operative disposition of structure 81, 82. This supporting and protecting comb structure described above provides especially good support for wispy or fragile plants, during cutting using rotating blades.

The pivotal or displaceable comb arrangement provided in cutting apparatus for use in micropropagation as described previously is of course also effective in retaining in position cut portions, whether the cutting takes place in the inverted position or in the normal upright disposition. The disadvantage however as compared with the removable comb arrangement now described is the greater constraint on ease of removal of the cut portions on completion of the cutting operation. By use of removable combs, the cut portions retained in the combs may be removed as an entity along with the combs, while still retained in the combs, to the planting-out location, this arrangement providing for ease of handling, whereas with the use of displaceable comb portions integrally associated with or pivotally connected to the cutting apparatus, it will normally be necessary for the cut portions to be released from the combs at the location of cutting, as the pivoting combs form part of the cutting equipment, thereby introducing an extra handling stage, with the possibility of loss or displacement of cuttings occasioned by the spillage inevitable during separation of cuttings from the comb portions at the cutting station. Thus while cutting in the inverted position offers certain advantages in terms of clearance of cuttings following the cutting operation where no combs are used or where displaceable but apparatus-mounted comb portions located at the cutting station are used, inversion of the plantlets or hedge for a single cutting stage in the method of the present invention is of less relevance in the event of use of freely locatable removable comb portions such as provided by the present invention for plant engagement during cutting, and is thus in no way essential in a single cutting stage as applied in the method of the present invention.

The present invention is thus particularly directed to the taking of a single layer of cuttings in any one cutting step of a micropropagation system or method involving cutting and regrowth of a "hedge". The hedge is then normally allowed to grow again to provide another top layer of apices before a further apex cutting operation is effected. Successive cuttings on a single hedge are not normally taken on the occasion of a particular cutting event. However, the necessity for multiple cuttings and also the desirability or otherwise of inversion in the cutting stage is also to an extent a function of the growth habit of the particular plants under cultivation. Potato plantlets are particularly suited to the arrangements described in detail in the foregoing sections, but alternative arrangements may be appropriate under a different growth habit or regime as associated with different types of plant.

Thus very large numbers of plants of a predetermined quality standard may be built up rapidly, typically over successive fourteen day growth periods, by initially establishing a multiplicity of hedges, and then cropping apex cuttings from these hedges to develop still further hedges. At a final stage in the procedure, the micro plantlets growing in hedges are severed to provide material for planting out, in a substantially normal external ambience, although typically protected from wind and chill by enclosure such as within a so-called tunnel. In this final cutting stage, the plantlets are not merely separated by an apex cutting operation, but the apex and a number of nodes are separated as a unit from the root structure of the plant remaining in the substrate. This relatively elongated cutting is then planted in soil or compost, with a number of the nodes below the surface of the soil, these being placed for the fostering of rapid root and tuber growth, which develops from the buried nodes.

Collection of the cuttings for subsequent placement, such as but not necessarily by manual handling, on the further growth medium facilitates accurate placement of the plantlets on the growth medium in question. Transfer of cut portions by their falling under gravity in a random manner onto a medium, such as a freshly gel led nutrient medium, in which they are left to root themselves, does not provide a subsequent hedge of appropriate uniformity and density for at least certain applications of micropropagation. Gravity collection of this kind with random self-induced replanting leads to an unacceptable variation in growth habit in at least some varieties of microplant. Thus a method in which a series of horizontal cuts are made through a mass of fairly straight stemmed plantlets growing from a gelled nutrient-medium to obtain cut crop parts containing on average at least one tip or node per cut portion, and, at each cut, the plant portions are transferred in bulk by for example air-flow or gravity to a fresh gelled nutrient medium, where they are planted by random scattering, is of limited utility for large scale micropropagation, such as is provided by the present invention. The methodology identified above may be acceptable for use with small plant quantities, to be received on media contained for example in petri dishes, and where growth takes place in heterotrophic manner on sucrose. It is not suited where photoautotrophic growth is in question. Applied to large quantities, random scattering also introduces intractable problems in respect of cutting orientation, and cutting overlap and overlay.

Plantlets of exterior origin may also be initially directly planted in a growth medium in a growth tray such as that of Figure 8, at relatively wide spacing, to provide a growing array of modest density. Cuttings taken from this original array may then be planted in a more dense arrangement for the next stage of cutting, to provide hedges of increasing density by repeated growing steps. Successive cuttings from successive hedges developing from original plants may be located at closer spacings. The particular methodology described above is especially suited to the development of micro potato plants.

A number of further features of the method of the invention may also be briefly mentioned. Environmental control of the ambience in the growth chamber within which the enclosed group plants, whether in agar or on foam, are accommodated, is also of importance in optimising growing conditions, to engender rapid and successful growth. The rate of growth may however be slowed down by reducing the temperature within the growth chamber in which manner a slower growth cycle may be achieved. This may in certain circumstances be advantageous, where for example a body of genetically certified material is required to be grown and recycled for preservation rather than multiplication, such as during the winter season. The mix of macro- and micro-nutrients used suitably follows preferred husbandry techniques, and may be adjusted as appropriate in both the agar and foam substrate growth phases, in accordance with the required conductivity of the nutrients. Control of the level of illumination of the growing plants, as well as the temperature of the growth chamber and the degree of carbon dioxide enrichment of the growth chamber, is suitably effected by known control techniques. The degree of carbon dioxide enrichment is relatively modest and may be contained and controlled within substantially conventional building structures. The racking or other support structures on which the growth enclosures or trays are supported are preferably of openwork structure, to provide for maximum freedom of gas flow and for maximum exposure of the growing enclosed plant trays to the high photo photon flux engendered by the illuminating arrangements.

The degree of permeability of the enclosing plastics material is not critical, but porosity must necessarily be present in all cases, for the passage of carbon dioxide and vapour as appropriate. In the preparation of apex and nodal cuttings for further growth, whether in the initial stages on agar or on the later stages of hedge growth on foam substrate, averaging of the cuttings is preferably practised, so that all cuttings when initially placed in agar or on foam are of substantially the same elongate extent. Thus a high quality product is achieved with a high degree of uniformity of growth dimensions. The use of the preferred foam as identified above provides for the variable density which is a preferred feature of the substrate used, and ensures that a reservoir for nutrient-containing liquid is established along the sides of the substrate structure, so that conductivity is higher away from the central region of the foam where plant growth initially occurs. The relatively open grain structure in the central region, provided by the characteristics of the foam and its subsequent mechanical perforation, ensure adequate moisture for the initial stages of plant growth, without plant damage engendered by the presence of an excess of concentration of nutrient, while still enabling plant growth to be further fomented as the root structure builds up and the roots extend out to the nutrient-containing reservoir regions at the sides of the substrate. While the particular substrate identified represents a preferred method of providing for plant growth in hedges, any alternative growth medium meeting the particular demands of plant growth in hedges may also be used. Preferred properties of the substrate are the ability to absorb water and to retain water, while also providing for the presence of a significant percentage volume of air to water, typically in excess of 20% at water tensions near to zero, thereby again facilitating root extension. Further requirements of the substrate medium are that it should retain at all times an appropriately located reservoir of nutrient solution of a strength sufficient to promote growth at all stages of plant development.

The nature of the enclosed environment in which hedge growth takes place together with the substrate provides less water in the centre of the substrate and more along its sides. A self-contained ecological environment is thus provided, in which the plants are exposed to initial high humidity to develop roots, but the humidity drops naturally according as plant development takes place, so that the plants then photosynthesise with natural root development, the plants thereby developing in a suitable manner for subsequent natural growth. The ambience established according to the method of the invention within the hedge enclosure provides the foregoing conditions in a substantially automatic manner, in conjunction with control of the external environment surrounding the enclosures. The liquid retentivity of the foam is sufficient to provide an adequate reservoir throughout the full growth period of the hedge, within the closed environment, by suitably charging the substrate before cutting placement and enclosure.

A further and final advantage of the foam arrangement particularly described in the present application is that the transverse compression of the foam, when it is engaged in a flanged base of the kind described, is such that when the tray is inverted, the foam substrate remains retained and does not fall out, even when it contains a substantial body of moisture.

## Claims

1. A method of micropropagation comprising the steps of:
(a) growing a plurality of plantlets (3, 4) on a first nutrient substrate medium (5),
(b) effecting a cutting operation on the plantlets (3, 4) at least at their apices to provide a plurality of apex cuttings (9) for assembly into an organised array for subsequent growth on a second substrate medium (11),
(c) enclosing at least some of said apex cuttings (9) and said second substrate medium (11) to define an enclosed region (13),
(d) exposing said enclosed region (13) to high intensity light and a carbon dioxide enriched external atmosphere, optionally with temperature control, for a period sufficient to secure a predetermined degree of microplant (14) growth within said enclosed region,
(e) supporting microplants (14) grown within said enclosed region (13), for a single cutting operation, in at least one flanged substantially rigid base structure (12) for accommodating a body (23) of substrate medium, said body (23) of substrate medium defining said second substrate medium (11), and
(f) dividing said microplants (14) in said single cutting operation to provide plantlet portions (15, 16) for further growth,
characterized in that
said body (23) of substrate medium defining said second substrate medium (11) has differential water and nutrient retention properties at different locations within said body (23) of substrate medium, and that the method also comprises the following further steps:
(i) forming said at least one flanged substantially rigid base structure (12) to comprise folded-in flanges or lips (33) extending along side edges of said base structure (12),
(ii) compressing edge regions of said body (23) of substrate medium to provide compressed edge regions (26, 27, 28) in which the density of the substrate medium is increased, and
(iii) receiving said compressed edge regions within and under said folded-in flanges or lips (33) of the base structure (12) for gripping engagement of the body (23) of substrate medium within said base structure (12).

2. A method according to Claim 1, wherein the dividing operation of step (f) provides plantlet portions (16), each of which comprises an apex (17) and a plurality of nodes (18) for planting out in a substantially natural environment for said further growth.

3. A method according to Claim 1, wherein the dividing operation of step (f) provides plantlet portions, each of which is an apex cutting (15), and said microplants (14) and said second substrate medium (11) are re-enclosed to again define said enclosed region (13) for steps corresponding to steps (d) through (f) of Claim 1.

4. A method according to Claim 3, wherein said apex cuttings (15) provided by the dividing operation of step (f) are used for subsequent growth and division in steps corresponding to steps (b) to (f) of Claim 1.

5. A method according to any preceding claim, wherein:
(1) said first nutrient substrate medium (5) comprises macro- and micro-nutrients gelled in agar,
(2) said cutting operation of stage (b) provides a plurality of nodal cuttings (8) and a plurality of apex cuttings (9), and
(3) at least some of said nodal cuttings (8) are placed in a further substrate medium comprising macro- and micro- nutrients gelled in agar for regrowth to provide further plantlets for use in a step corresponding to step (a) of Claim 1.

6. A method according to any preceding claim, wherein the cuttings are assembled for said subsequent growth on a structured low density expanded polyurethane foam defining said substrate medium (11) having differential water and nutrient retention properties.

7. Apparatus for effecting a dividing operation in a method of micropropagation in which:
(a) a plurality of plantlets (3, 4) are grown on a first nutrient substrate medium (5),
(b) a cutting operation is effected on the plantlets (3, 4) at least at their apices to provide a plurality of apex cuttings (9) for subsequent growth on a second substrate medium (11),
(c) at least some of said apex cuttings (9) and said second substrate medium (11) are enclosed to define an enclosed region (13),
(d) said enclosed region (13) is exposed to high intensity light, optionally with temperature control, for a period sufficient to secure a predetermined degree of microplant growth (14) within said enclosed region (13), and
(e) microplants (14) grown within said enclosed region (13) are divided in a single cutting operation to provide plantlet portions (15, 16) for further growth,
the apparatus comprising:
(1) a cutting head (62),
(2) means (12) for supporting said microplants (14) for said single cutting operation in said dividing step (e), and
(3) means (64, 65) for effecting displacement of said cutting head (62) relative to said microplants (14),
wherein said means for supporting said microplants (14) for said single cutting operation comprises at least one flanged substantially rigid base structure (12) accommodating a body (23) of substrate medium, said body (23) of substrate medium defining said second substrate medium (11),
characterized in that
(i) said at least one flanged substantially rigid base structure (12) comprises folded-in flanges or lips (33) extending along side edges of said base structure (12),
(ii) said body (23) of substrate medium has differential water and nutrient retention properties at different locations within said body (23) of substrate medium,
(iii) said body (23) of substrate medium has compressed edge regions (26, 27, 28) in which the density of the substrate medium is increased, and
(iv) said compressed edge regions are received within and under said folded-in flanges or lips (33) of the base structure (12) for gripping engagement of the body (23) of substrate medium within said base structure (12).

8. Apparatus according to Claim 7, comprising means (67, 81, 82) for constraining said microplants (14) against significant displacement in at least one direction during said single cutting operation.

9. Apparatus according to Claim 8, wherein said constraining means includes at least two toothed comb-like structures (71, 81, 82) for engaging portions of plant material forming an array of said microplants (14) from opposite sides of said array.

10. Apparatus according to Claim 9, wherein each of said comb-like structures (71, 81, 82) is displaceably mounted (67, 87) for movement between array-constraining and array-release dispositions.

11. Apparatus according to Claim 9, wherein each of said comb-like structures (71, 81, 82) is an independent component for placement in an array-constraining disposition prior to said single cutting operation.

## Patentansprüche

1. Verfahren der Mikorvermehrung, das die folgenden Schritte umfaßt:
a) Ziehen einer Vielzahl von Pflänzchen (3,4) auf einem ersten Nährsubstratmedium (5),
b) Ausführen eines Schneidvorgangs an den Pflänzchen (3,4) wenigstens an ihren Spitzen, um eine Vielzahl von Spitzensetzlingen (9) herzustellen, die in organisierter Anordnung zum anschließenden Wachstum auf einem zweiten Substratmedium (11) angeordnet werden,
c) Umschließen wenigstens einiger der Spitzensetzlinge (9) und des zweiten Substratmediums (11), so daß ein umschlossener Bereich (13) entsteht,
d) Einwirken von Licht hoher Intensität und einer mit Kohlendioxid angereicherten äußeren Atmosphäre, die wahlweise temperaturgesteuert wird, auf den umschlossenen Bereich (13) über einen Zeitraum, der ausreicht, um einen vorgegebenen Grad des Wachstums von Jungpflanzen (14) in dem umschlossenen Bereich sicherzustellen,
e) Aufnehmen von Jungpflanzen (14), die in dem umschlossenen Bereich (13) gezogen wurden, für einen einzelnen Schneidvorgang in wenigstens einer mit Flansch versehenen, im wesentlichen starren Trägerstruktur (12), die einen Korper (23) aus Substratmedium aufnimmt, wobei der Körper (23) aus Substratmedium das zweite Substratmedium (11) bildet, und
f) Teilen der Jungpflanzen (14) in einem einzelnen Schneidvorgang, um Pflänzchenteile (15,16) für das weitere Wachstum herzustellen,
**dadurch gekennzeichnet, daß:**
der Körper (23) aus Substratmedium, der das zweite Substratmedium (11) bildet, unterschiedliche Wasser- und Nährstoffhalteeigenschaften an verschiedenen Stellen in dem Körper (23) aus Substratmedium aufweist, und daß das Verfahren darüber hinaus die folgenden weiteren Schritte umfaßt:
1. Formen der wenigstens einen mit Flansch versehenen, im wesentlichen starren Trägerstruktur (12), so daß sie nach innen gefaltete Flansche bzw. Ränder (33) umfaßt, die sich an Seitenkanten der Trägerstruktur (12) entlang erstrecken,
2. Zusammendrücken von Kantenbereichen des Körpers (23) aus Substratmedium, um zusammengedrückte Kantenbereiche (26,27,28) herzustellen, in denen die Dichte des Substratmediums größer ist, und
3. Aufnehmen der zusammengedrückten Randbereiche in und unter den nach innen gefalteten Flanschen bzw. Rändern (33) der Trägerstruktur (12), um haltenden Eingriff des Körpers (23) aus Substratmedium in der Trägerstruktur (12) herzustellen.

2. Verfahren nach Anspruch 1, wobei mit dem Teilvorgang in Schritt f) Pflänzchenteile (16) erzeugt werden, die jeweils eine Spitze (17) und eine Vielzahl von Knoten (18) umfassen und zum weiteren Wachstum in eine im wesentlichen natürliche Umgebung ausgepflanzt werden.

3. Verfahren nach Anspruch 1, wobei bei dem Teilvorgang in Schritt f) Pflänzchenteile entstehen, bei denen es sich jeweils um einen Spitzensetzling (15) handelt, und die Jungpflanzen (14) und das zweite Substratmedium (16) wieder umschlossen werden, um wiederum den umschlossenen Bereich (13) für Schritte entsprechend den Schritten d) bis f) von Anspruch 1 zu bilden.

4. Verfahren nach Anspruch 3, wobei die Spitzensetzlinge (15), die durch den Teilvorgang in Schritt f) hergestellt werden, für das anschließende Wachstum und das Teilen in Schritten eingesetzt werden, die den Schritten b) bis f) gemäß Anspruch 1 entsprechen.

5. Verfahren nach einem der vorangehenden Ansprüche, wobei:
1. das erste Nährsubstratmedium (5) Makro- und Mikronährstoffe enthält, die in Agar geliert sind,
2. durch den Schneidvorgang von Stufe b) eine Vielzahl von Knotensetzlingen (8) und eine Vielzahl von Spitzensetzlingen (9) entstehen, und
3. wenigstens einige der Knotensetzlinge (8) in ein weiteres Substratmedium gegeben werden, das Makro- und Mikronährstoffe enthält, die in Agar geliert sind. um erneut zu wachsen und weitere Pflänzchen zum Einsatz in einem Schritt herzustellen, der Schritt a) von Anspruch 1 entspricht.

6. Verfahren nach einem der vorangehenden Ansprüche, wobei die Setzlinge zum anschließenden Wachstum auf einem strukturierten Polyurethanschaum niedriger Dichte angeordnet werden, der das Substratmedium (11) mit unterschiedlichen Wasser- und Nährstoffhalteeigenschaften bildet.

7. Vorrichtung zum Ausführen eines Teilvorgangs bei einem Verfahren der Mikrovermehrung, bei dem:
a) eine Vielzahl von Pflänzchen (3,4) auf einem ersten Nährsubstratmedium (5) gezogen werden,
b) ein Schneidvorgang an den Pflanzchen (3,4) wenigstens an ihren Spitzen ausgeführt wird, um eine Vielzahl von Spitzensetzlingen (9) zum anschließenden Wachstum auf einem zweiten Substratmedium (11) herzustellen.
c) wenigstens einige der Spitzensetzlinge (9) und das zweite Substratmedium (11) umschlossen werden, um einen umschlossenen Bereich (13) zu bilden,
d) der umschlossene Bereich (13) Licht hoher Intensität wahlweise mit Temperatursteuerung, über einen Zeitraum ausgesetzt wird, der ausreicht, um einen vorgegebenen Grad des Jungpflanzenwachstums (14) in dem umschlossenen Bereich (13) sicherzustellen, und
e) Jungpflanzen (14), die in dem umschlossenen Bereich (13) gezogen wurden, in einem einzelnen Schneidvorgang geteilt werden, um Pflänzchensetzlinge (15) zum weiteren Wachstum herzustellen,
wobei die Vorrichtung umfaßt:
1. einen Schneidkopf (60)
2. eine Einrichtung (12), die die Jungpflanzen (14) bei dem einzelnen Schneidvorgang in dem Teilschritt (e) aufnimmt, und
3. Einrichtungen (64,65), die Verschiebung des Schneidkopfes (62) in bezug auf die Jungpflanzen (14) bewirken,
wobei die Einrichtung, die die Jungpflanzen (14) bei dem einzelnen Schneidvorgang aufnimmt, wenigstens eine mit Flansch versehene, im wesentlichen starre Trägerstruktur (12) umfaßt, die einen Körper (23) aus Substratmedium aufnimmt, wobei der Körper (23) aus Substratmedium das zweite Substratmedium (11) bildet,
**dadurch gekennzeichnet, daß:**
1. die wenigstens eine mit Flansch versehene, im wesentlichen starre Trägerstruktur (12) nach innen gefaltete Flansche bzw. Ränder (33) umfaßt, die sich an Seitenkanten der Trägerstruktur (12) entlang erstrecken,
2. der Körper (23) aus Substratmedium unterschiedliche Wasser- und Nährstoffhalteeigenschaften an verschiedenen Stellen in dem Körper (23) aus Substratmedium hat,
3. der Körper (23) aus Substratmedium zusammengedrückte Kantenbereiche (26,27,28) hat, in denen die Dichte des Substratmediums größer ist, und
4. die zusammengedrückten Kantenbereiche in und unter den nach innen gefalteten Flanschen bzw. Rändem (33) der Trägerstruktur (12) aufgenommen werden, um haltenden Eingriff des Körpers (23) aus Substratmedium in der Trägerstruktur (12) herzustellen.

8. Vorrichtung nach Anspruch 7, die Einrichtungen (67,81,82) umfaßt, die Jungpflanzen (14) an nennenswerter Verschiebung in wenigstens einer Richtung während des einzelnen Schneidvorgangs hindern.

9. Vorrichtung nach Anspruch 8, wobei die Hinderungseinrichtung wenigstens zwei gezahnte kammartige Strukturen (71,81,82) enthält, die mit Teilen von Pflanzenmaterial, das eine Anordnung der Jungpflanzen (14) bildet, von einander gegenüberliegenden Seiten der Anordnung her in Kontakt kommen.

10. Vorrichtung nach Anspruch 9, wobei jede der kammartigen Strukturen (71,81,82) verschiebbar (67,87) angebracht ist, so daß sie sich zwischen den die Anordnung hindernden und die Anordnung freigebenden Positionen bewegen können,

11. Vorrichtung nach Anspruch 9, wobei jede der kammartigen Strukturen (71,81,82) unabhängiges Bauteil ist, das vor dem einzelnen Schneidvorgang in eine die Anordnung hindernde Position gebracht werden kann.

## Revendications

1. Procédé de microreproduction comprenant les étapes consistant à:
(a) faire croître une pluralité de plantules (3, 4) sur un premier substrat nutritif (5),
(b) effectuer une opération de coupe sur les plantules (3, 4) au moins au niveau de leurs têtes pour obtenir une pluralité, de boutures de tête (9) destinées à être assemblées en une rangée organisée en vue d'une croissance subséquente sur un deuxième substrat (11).
(c) enfermer au moins certaines desdits boutures (9) et ledit deuxième substrat (11) pour former une région close (13).
(d) exposer ladite région close (13) a un lumière de forte intensité et à une atmosphère extérieure enrichie en gaz carbonique, facultativement en contrôlant la température, pendant une période de temps suffisante pour assurer un degré prédéterminé de croissance de microplants (14) dans ladite région close.
(e) supporter, pendant une opération de coupe unique, les microplants (14) ayant grandi à l'intérieur de ladite région close (13), dans au moins une structure de base (12) sensiblement rigide et pourvue de rebords, destinée à loger un corps (23) de substrat, ledit corps (23) de substrat formant ledit deuxième substrat (11), et
(f) diviser lesdits microplants (14), au cours de ladite opération de coupe unique, pour obtenir des parties de plantules (15, 16) en vue d'une autre croissance,
caractérisé en ce que:
ledit corps (23) de substrat formant ledit deuxième substrat (11) possède des propriétes de rétention différentielle d'eau et de substances nutritives en différents endroits dans ledit corps (23) de substrat, et en ce que le procédé comprend aussi les étapes supplémentaires suivantes consistant à:
(i) former ladite structure de base (12), sensiblement rigide et pourvue de rebords, de manière qu'elle comprenne des rebords ou lèvres rabattables (33) s'étendant le long des bords latéraux de ladite structure de base (12).,
(ii) comprimer des régions marginales dudit corps (23) de substrat pour obtenir des régions marginales comprimées (27, 27, 28) dans lesquelles la densité du substrat se trouve accrue, et
(iii) recevoir lesdites régions marginales comprimées dans les limites et sous lesdits rebords on lèvres rabattables (33) de la structure de base (12) pour enserrer le corps (23) de substrat à l'intérieur de ladite structure de base (12).

2. Procédé selon la revendication 1, dans lequel l'opération de division de l'étape (f) procure des parties de plantules (16), dont chacune comprend une tête (17) et une pluralité de noeuds (18), en vue d'une plantation à l'extérieur dans un environnement sensiblement naturel pour ladite autre croissance supplémentaire.

3. Procédé selon la revendication 1, dans lequel l'opération de division de l'étape (f) procure des parties de plantules, dont chacune est une bouture de tête (15), et lesdits microplants (14) et le deuxième substrat (11) sont ré-enfermés pour former de nouveau ladite région close (13) pendant des étapes correspondant aux étapes (d) à (f) de la revendication 1.

4. Procédé selon la revendication 3, dans lequel lesdites boutures (15) obtenues par l'opération de division de l'étape (f) sont utilisées pour une croissance et une division ultérieures au cours d'étapes correspondant aux étapes (b) à (f) de la revendication 1.

5. Procédé selon l'une quelconque des revendications précédentes, dans lequel:
(1) ledit premier substrat nutritif (5) comprend des macro- et microsubstances nutritives dans un gel d'agar-agar;
(2) ladite opération de coupe de l'étape (b) procure une pluralité de boutures nodales (8) et une pluralité de boutures de tête (9), et
(3) au moins certaines desdites boutures nodales (8) sont placées dans un autre substrat comprenant des macro- et microsubstances nutritives dans un gel d'agar-agar en vue d'une nouvelle croissance pour obtenir d'autres plantules destinées à être utilisées pendant une étape correspondant à l'étape (11) de la revendication 1.

6. Procédé selon l'une quelconque des revendications précédentes, dans lequel les boutures sont assemblées en vue de ladite croissance subséquente sur une mousse structurée de polyuréthane expansé de faible densité formant ledit substrat (11) possédant des propriétés de rétention différentielle d'eau et de substances nutritives.

7. Appareil permettant d'effectuer une opération de division dans un procédé de microreproduction dans lequel:
(a) on fait croître une pluralité de plantules (3, 4) sur un premier substrat nutritif (5),
(b) on effectue une opération de coupe sur les plantules (3, 4) au moins au niveau de leurs têtes pour obtenir une pluralité de boutures de tête (9) en vue d'une croissance subséquente sur un deuxième substrat (11),
(c) on enferme au moins certaines desdites boutures de tête (9) et ledit deuxième substrat (11) pour former une région close (13),
(d) on expose ladite région close (13) à un lumière de forte intensité, facultativement en contrôlant la température, pendant une période de temps suffisante pour assurer un degré prédéterminé de croissance de microplants (14) dans ladite région close (13),
(e) on divise, au cours d'une opération de coupe unique, les microplants (14) ayant grandi dans ladite région close (13) pour obtenir des parties de plantules (15, 16) en vue d'une autre croissance,
l'appareil comprenant:
(1) une tête de coupe (62),
(2) un moyen (12) destiné à supporter lesdites microplants (14) pour ladite opération de coupe unique de ladite étape de division (e), et
(3) un moyen (64, 65) destiné à effectuer un déplacement de ladite tête de coupe (62) par rapport auxdits microplants (14),
ledit moyen destiné à supporter lesdits microplants (14) pendant ladite opération de coupe unique comprenant au moins une structure de base (12) sensiblement rigide et pourvue de rebords logeant un corps (23) de substrat formant ledit deuxième substrat (11),
caractérisé en ce que
(i) ladite structure de base (12) sensiblement rigide et pourvue de rebords comprend des rebords ou lèvres rabattables (33) s'étendant le long des bords latéraux de ladite structure de base (12),
(ii) ledit corps (23) du substrat possède des propriétés de rétention différentielle d'eau et de substances nutritives en différents endroit dans ledit corps (23) du substrat,
(iii) ledit corps (23) du substrat comporte des régions marginales (26, 27, 28) dans lesquelles la densité du substrat est accrue, et
(iv) lesdites régions marginales comprimées sont reçues dans les limites et sous lesdits rebords ou lèvres rabattables (33) de ladite structure de base (12) en vue d'un enserrement du corps (23) de substrat se trouvant dans ladite structure de base (12).

8. Appareil selon la revendication 7, comprenant un moyen (67, 81, 82) destiné à retenir lesdits microplants (14) à l'encontre de tout déplacement important dans au moins une direction pendant ladite opération de coupe unique.

9. Appareil selon la revendication 8, dans lequel ledit moyen de retenue comprend au moins deux structures dentées en forme de peignes (71, 81, 82) destinées a venir en prise avec des parties des plants en formant des rangs desdits microplants (14) depuis les côtés opposés desdits rangs.

10. Appareil selon la revendication 9, dans lequel chacune desdites structures (71, 81, 82) est montée de façon mobile (67, 87) en vue d'un déplacement entre des dispositions de maintien de rangs et de relâchement de rangs.

11. Appareil selon la revendication 9, dans lequel chacune desdites structures en forme de peignes (71, 81, 82) est un composant indépendant destiné à être placé dans une disposition de maintien de rangs avant ladite opération de coupe unique.
